# EUROPEAN PATENT SPECIFICATION

(11) **EP 3 260 528 B1**
(45) Date of publication and mention of the grant of the patent: **10.06.2020**
(21) Application number: 16772146.3
(22) Date of filing: 09.03.2016
(51) Int. Cl.: C12M 1/00, C12M 3/00, C12M 1/12

(54) **CELL CULTURE APPARATUS**
ZELLKULTURVORRICHTUNG
APPAREIL DE CULTURE CELLULAIRE

(30) Priority: 31.03.2015 JP 2015071448
(43) Date of publication of application: 27.12.2017
(73) Proprietor: PHC Holdings Corporation, Tokyo (JP)
(72) Inventor: BABA, Manami, Ehime 791-0395 (JP); TOKUMARU, Tomoyoshi, Ehime 791-0395 (JP)
(74) Representative: Grünecker Patent- und Rechtsanwälte PartG mbB
(86) International application number: PCT/JP2016/057295
(87) International publication number: WO 2016/158262

(56) References cited:
- EP-A1- 2 180 037
- WO-A1-2005/046401
- JP-A- 2001 309 836
- JP-A- 2005 195 769
- JP-A- 2007 020 964
- JP-A- 2007 054 144
- JP-A- 2007 139 891
- JP-A- 2012 120 740
- JP-A- 2014 062 237
- JP-U- 3 112 237
- DATABASE WPI Week 200609 Thomson Scientific, London, GB; AN 2006-083862 XP002777552, & JP 2006 014675 A (J TECH KK) 19 January 2006 (2006-01-19)
- DATABASE WPI Week 200408 Thomson Scientific, London, GB; AN 2004-075905 XP002777553, & JP 2004 000275 A (SANYO ELECTRIC CO LTD) 8 January 2004 (2004-01-08)
- 'C02 Incubator', [Online] December 2013, XP055320386 Retrieved from the Internet: <URL:http://www.k-okuma.co.jp/PDF/KM-CC17.p df> [retrieved on 2015-03-29]

## Description

### [Technical Field]

The present disclosure relates to a culture apparatus.

### [Background Art]

In culture apparatuses, such a structure is known as an example in which stainless steel shelves for placement of cultures are supported by shelf rests.

### [Citation List]

### [Patent Literature]

[Patent Literature 1] Japanese Patent Application Publication No. 2010-17151

### [Summary of Invention]

### [Technical Problem]

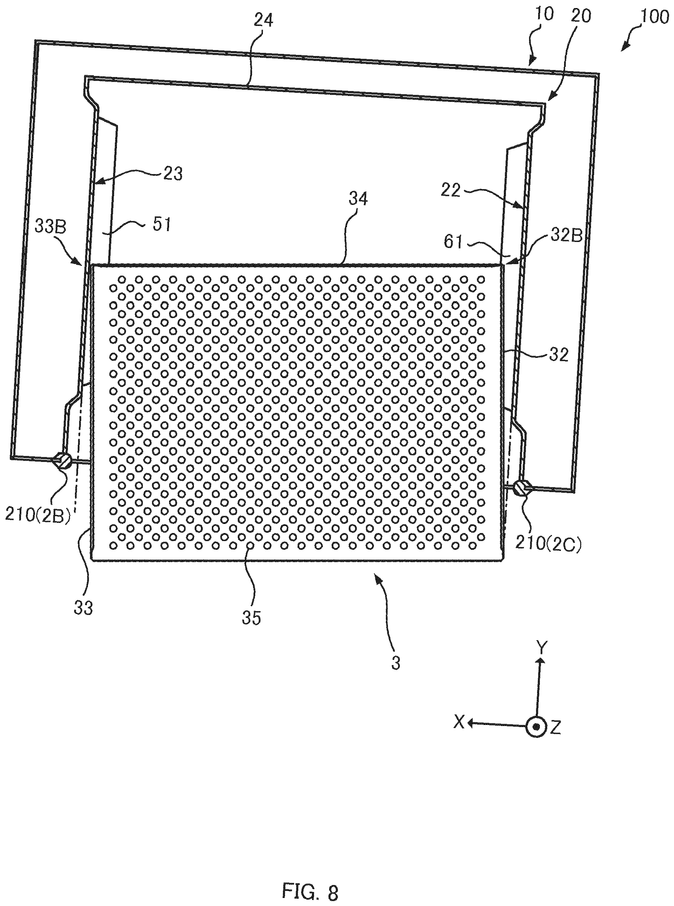

In conventional culture apparatuses, after hydrogen peroxide decontamination, dry heat sterilization, an operation in a non-humidified state, or the like, there are cases where the shelf plates slide less smoothly, and thus the shelf plates are inserted and withdrawn less easily. EP2180037 discloses a (claim 1) a shelf plate provided in the culture chamber. The shelves may be made of stainless steel [0014]. The shelves are bent on the sides (fig. 8, side pieces 432).

In view of this, an aspect of the present disclosure is to provide a culture apparatus in which a shelf plate can slide easily.

### [Solution to Problem]

A main disclosure to solve an issue described above is a culture apparatus including the features of claim 1.

### [Advantageous Effects]

As described above, the present disclosure makes it possible to provide a culture apparatus in which a coefficient of friction is reduced as compared with a coefficient of friction when the shelf plate contacts a member made of the same material; sliding of the shelf plate is facilitated; and insertion and withdrawal thereof are facilitated, so that a shelf can slide easily.

### [Brief Description of the Drawings]

FIG. 1 is an example of a perspective view illustrating a culture apparatus according to a first embodiment.
FIG. 2 is an example of a perspective view illustrating a culture apparatus in a state where an outer door and an inner door are open according to a first embodiment.
FIG. 3 is an example of a cross-sectional view illustrating an inner case according to a first embodiment.
FIG. 4 is an example of a perspective view illustrating a shelf plate according to a first embodiment as viewed from above.
FIG. 5 is an example of a perspective view illustrating a shelf plate according to a first embodiment as viewed from below.
FIG. 6 is an example of an enlarged perspective view illustrating end portions of a back surface flange and a side surface flange in FIG. 5.
FIG. 7 is an example of an enlarged cross-sectional view illustrating a contact portion between a shelf plate and a shelf rest according to a first embodiment.
FIG. 8 is an example of a top view illustrating a case where a shelf plate has not been inserted or withdrawn in parallel to shelf rests according to a first embodiment.
FIG. 9 is a side view illustrating a relationship between a shelf plate and a side surface of an inner case according to a first embodiment.
FIG. 10 is an example of a perspective view illustrating a sliding member in another embodiment.
FIG. 11 is an example of an enlarged perspective view illustrating end portions of a back surface flange and a side surface flange attached with the sliding member according to another embodiment.
FIG. 12 is an example of an enlarged top view illustrating end portions of a back surface flange and a side surface flange attached with the sliding member according to another embodiment.
FIG. 13 is an example of an enlarged cross-sectional view illustrating a portion of contact between a shelf plate and a shelf rest according to another embodiment.

### [Description of Embodiments]

Hereinafter, embodiments will be described in detail with reference to the drawings as necessary. It should be noted that unnecessarily detailed descriptions may be omitted in some cases. For example, detailed descriptions for well-known matters may be omitted, and redundant descriptions for substantially the same configurations may be omitted. This is to avoid unnecessary redundancy in the below description, and to facilitate understanding by a person skilled in the art.

It should be noted that the inventors have provided the accompanying drawings and below description in order for a person skilled in the art to sufficiently understand the present disclosure, but not to limit the subject matter recited in the claims.

### First Embodiment

### Culture apparatus

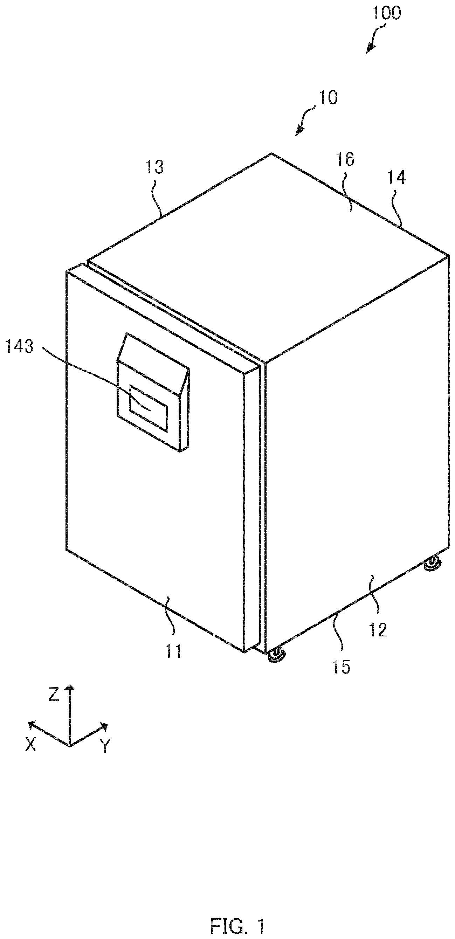

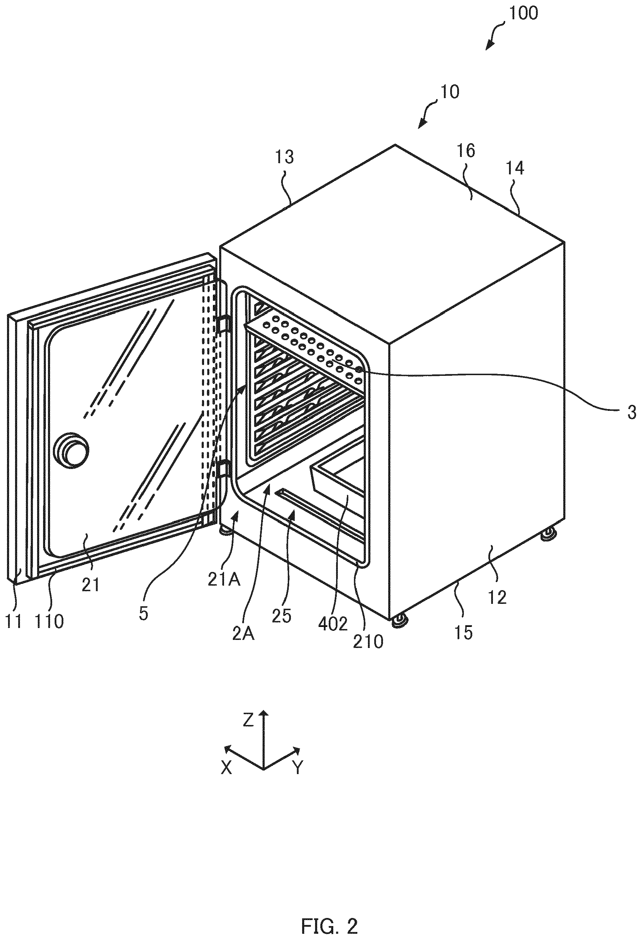

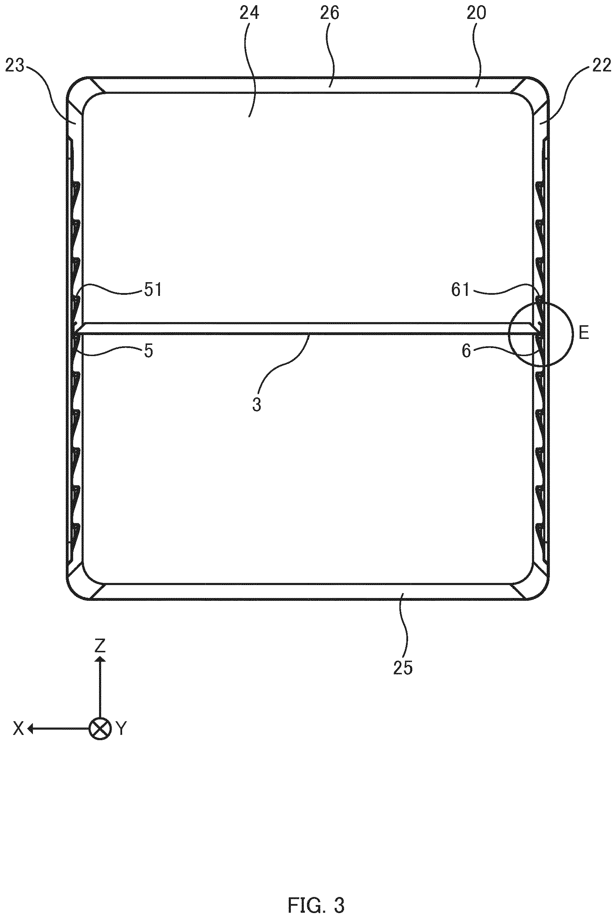

Hereinafter, a culture apparatus 100 according to an embodiment of the present disclosure will be described as an example of a culture apparatus according to the present disclosure, with reference to FIGS. 1 to 9. FIG. 1 is a perspective view illustrating the culture apparatus 100 according to an embodiment of the present disclosure. FIG. 2 is a perspective view illustrating the culture apparatus 100 in the state where an outer door and an inner door are open according to an embodiment of the present disclosure. FIG. 3 is a cross-sectional view illustrating an inner case 20 of an embodiment of the present disclosure. FIG. 3 illustrates the culture apparatus 100 when viewed toward the +X direction from a cross-section, parallel to the ZY plane, passing through substantially the center of the culture apparatus 100.

It should be noted that the X axis is the axis that is orthogonal to side plates 12 and 13 of an outer case 10, where +X is the direction from the side plate 12 toward the side plate 13, and -X is the direction from the side plate 13 toward the side plate 12. The Y axis is the axis that is orthogonal to an outer door 11 and a back plate 14, where +Y is the direction from the outer door 11 toward the back plate 14, and -Y is the direction from the back plate 14 toward the outer door 11. The Z axis is the axis that is orthogonal to a bottom plate 15 and a top plate 16, where +Z is the upward direction from the bottom plate 15 toward the top plate 16, and -Z is the downward direction from the top plate 16 toward the bottom plate 15.

The culture apparatus 100 is an apparatus configured to cultivate cultures such as cells or microorganisms in a culture chamber 2A. The culture apparatus 100 has an outer case 10, an outer door 11, an inner case 20, an inner door 21, a humidification tray 402, and a shelf plate 3.

The outer case 10 and the inner case 20 each are a substantially rectangular parallelepiped box body that is made of a metal material such as stainless steel. The interior of the culture chamber 2A is sterilized using hydrogen peroxide water or the like, and therefore a material that is resistant to hydrogen peroxide and is also antibacterial is selected as a material constituting the inner case 20. The inner case 20 has an external form smaller than an internal form of the outer case 10, such that the inner case 20 is housed inside the outer case 10. An opening 21A leading to the culture chamber 2A is formed in a front surface of the outer case 10 and the inner case 20.

The outer door 11 and the inner door 21 are doors configured to open/close the opening 21A. The outer door 11 is made of a metal material such as stainless steel, is of a substantially rectangular shape having a larger outer form as compared with the inner door 21, and is filled with thermal insulation in its interior. A gasket 110 to ensure airtightness inside the culture apparatus 100 is provided along the peripheral edge of the outer door 11 on the side adjacent to the inner door 21. An operating device 143 configured to operate the culture apparatus 100 is provided on the outer door 11 on the side opposite to the inner door 21. The inner door 21 is a member made of a transparent material such as resin or glass. A gasket 210 to ensure airtightness inside the culture apparatus 100 is provided to a portion, to face and contact the inner door 21, along the edge of the opening 21A in the front surface of the outer case 10.

The inner case 20 includes side plates 22 and 23, a back plate 24, a bottom plate 25, and a top plate 26. A plurality of shelf rests 5 and 6 are provided to the side plates 22 and 23 by performing press work. The shelf plate 3 is placed on the shelf rests 5 and 6. The shelf rests 5 are respectively paired with the shelf rests 6 and such a pair supports a shelf plate 3 so that the shelf plate 3 is substantially horizontal.

The shelf rests 5 each protrude in a direction away from the side plate 13 of the outer case 10, and have an elongated shape to continuously range from the inner door 21 side (-Y) to the back plate 24 side (+Y) . The shelf rests 5 each have a placement surface 51. Similarly, the shelf rests 6 also each protrude in a direction away from the side plate 12 of the outer case 10, and have an elongated shape to continuously range from the inner door 21 side (-Y) to the back plate 24 side (+Y). The shelf rests 6 each have a placement surface 61.

The humidification tray 402 is a container in which water generated when humidifying the culture chamber 2A is stored, and is placed on the bottom plate 25 of the inner case 20.

The shelf plate 3 has a substantially rectangular shape and is made of a metal material such as stainless steel, and is placed on the shelf rests 5 and 6 that are provided to the inner case 20. The interior of the culture chamber 2A is sterilized using hydrogen peroxide water or the like, and therefore a material that is antibacterial as well as resistant to hydrogen peroxide is selected as the material constituting the shelf plate 3. In an embodiment of the present disclosure, the same material as that of the inner case 20 is selected as a material constituting the shelf plate 3. It should be noted that one single shelf plate 3 may be provided in the inner case 20, or multiple shelf plates 3 may be provided. The detailed configuration of the shelf plate 3 will be described later.

### Shelf plate

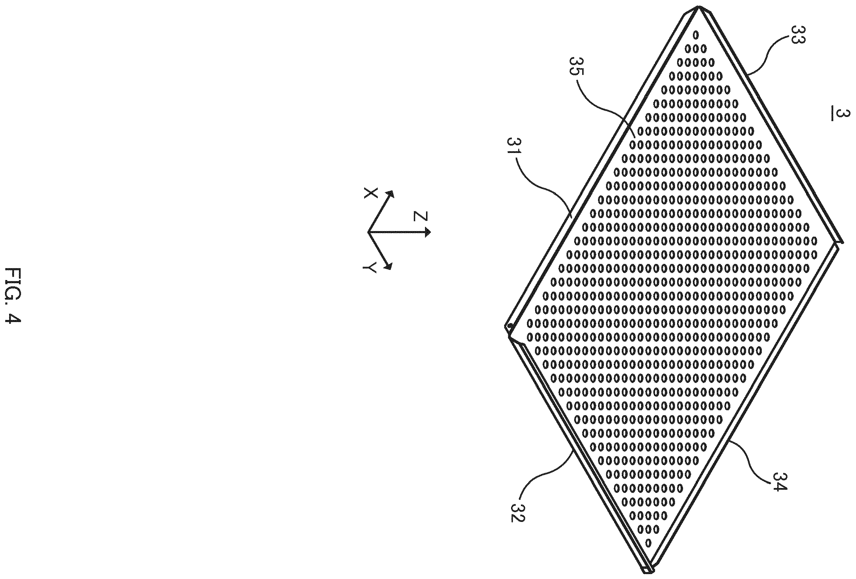

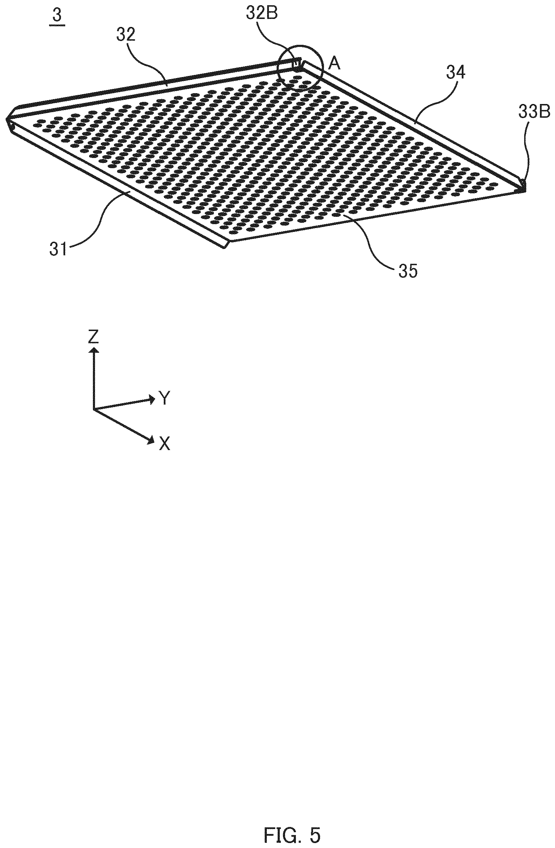

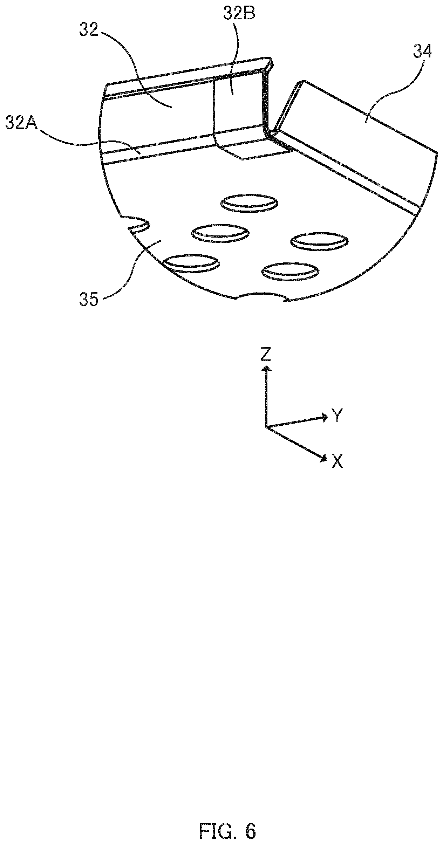

Hereinafter, the shelf plate according to an embodiment of the present disclosure will be described with reference to FIGS. 4 to 6. FIG. 4 is a perspective view illustrating the shelf plate 3 according to an embodiment of the present disclosure as viewed from above. FIG. 5 is a perspective view illustrating the shelf plate 3 according to an embodiment of the present disclosure as viewed from below. FIG. 6 is an enlarged perspective view illustrating end portions of a back surface flange 34 and a side surface flange 32 of FIG. 5.

The shelf plate 3 has a base surface 35 for the placement of cultures, and flanges 32 and 33 that are formed by being bent upward at bent portions 32A and 33A (not shown) in end portions on the left and right sides (+X and -X) of the base surface 35. The shelf plate 3 has a flange 34 that is formed by being bent upward at an end portion on the back surface side (+Y) of the base surface 35. The shelf plate 3 has a flange 31 that is formed by being bent downward at an end portion on the front surface side (-Y) of the base surface 31. The base surface 35 is a portion that is substantially parallel with the horizontal direction in the state where the shelf plate 3 is placed on the shelf rests 5 and 6. The flanges 31 to 34 are portions that are substantially parallel with the vertical direction in the state where the shelf plate 3 is placed on the shelf rests 5 and 6. In other words, the flanges 31 to 34 are portions that are substantially orthogonal to the base surface 35. The bent portions 32A and 33A are bent portions that connect the flanges 32, 33 and the base surface 35. It should be noted that the upper end portions of the flanges 32 and 33 each are provided with a portion that is folded further inward.

Sliding members 32B and 33B, which are made of a material different from the material of the inner case 20, are provided on portions of the two ends of the shelf plate 3 on the back surface side (+Y side), and extend from the outer surfaces of the flanges 32 and 33 to the lower surface of the base surface 35. A fluorine coating is used as the sliding members 32B and 33B. The sliding members 32B and 33B are applied, in a range of approximately 10.0 mm from the end portion on the back surface side (+Y) of the outer surfaces of the flanges 32 and 33 on the left and right sides, the outer surfaces of the bent portions 32A and 33A, and the lower surface of the base surface 35. On the outer surfaces of the flanges 32 and 33 on the left and right sides, the sliding members 32B and 33B are applied in a range from the upper end portion (+Z) to the lower end portion (-Z). On the lower surface of the base surface 35, the sliding members 32B and 33B are applied over a range of approximately 10.0 mm from the two end portions in the left-right direction (+X and -X) of the shelf plate 3.

### Relationship between shelf plate and shelf rests

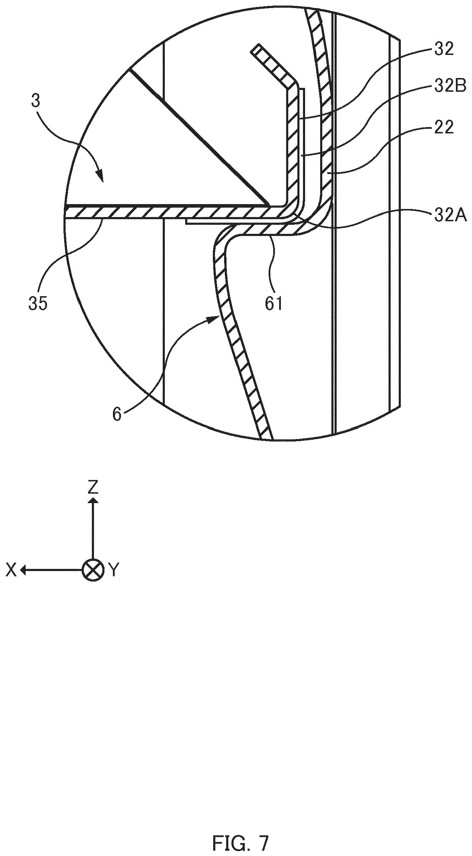

Hereinafter, the relationship between the shelf plate 3 and the shelf rests 5 and 6 according to an embodiment of the present disclosure will be described with reference to FIG. 7. FIG. 7 is an enlarged cross-sectional view of a portion of contact between the shelf plate 3 and the shelf rest 6. The following describes the relationship between the shelf plate 3 and the shelf rest 6 on the flange 32 side (-X) of the shelf plate 3. The relationship between the shelf plate 3 and the shelf rest 5 on the flange 33 side (+X) is similar and thus will be partially omitted.

The dimensions of the shelf plate 3 in the left-right direction are designed such that, when the shelf plate 3 is placed in the center between the left and right shelf rests 5 and 6, the respective distances from the flanges 32 and 33 to the side surfaces 22 and 23 of the inner case are approximately 2.0 mm. For this reason, as shown in FIG. 7, the shelf plate 3 is placed on the shelf rest 6 such that the lower surface of the base surface 35 comes into contact with the placement surface 61 of the shelf rest 6. In the state where the shelf plate 3 is placed in the center between the left and right shelf rests 5 and 6, the flange 32 does not come into contact with the side surface 22 of the inner case. The sliding member 32B is applied so as to cover the flange 32, the bent portion 32A, and the base side surface 35. Thus, the sliding member 32B and the placement surface 61 of the shelf rest 6 come into contact with each other in the region, of the shelf plate 3, provided with the sliding member 32B. Accordingly, frictional resistance at such a contact surface of the base surface 35 and the shelf rest 6 decreases, thereby facilitating sliding of the shelf plate 3. Also, when the shelf plate 3 is moved and the flange 32 comes into contact with the side surface 22 of the inner case, the sliding member 32B and the side surface 22 come into contact with each other. Accordingly, frictional resistance at the contact surface between the flange 32 and the side surface 22 decreases, thereby facilitating sliding of the shelf plate 3.

Also, a curved portion is provided in the portion connecting the side surface 22 of the inner case and the placement surface 61 of the shelf rest 6 since the shelf rest 6 is formed by press work, and the bent portion 32A of the shelf plate 3 may come into contact with this curved portion. In this case as well, frictional resistance can be reduced due to the sliding member 32B applied to the bent portion 32A. For this reason, not only sliding of the shelf plate 3 is facilitated, but also the shelf plate 3 becomes substantially centered in the left-right direction, and the shelf plate 3 becomes horizontal.

Also, since the shelf rest 6 is formed by press work, the placement surface 61 maybe slightly inclined. Specifically, there are cases where the placement surface 51 may be inclined toward the bottom plate 25 of the inner case 20 as distance, in the direction (+X), from the side plate 22 of the inner case 20. In this case, the shelf rest 6 and the bent portion 32A come into linear contact with each other, but sliding of the shelf plate 3 is facilitated due to the sliding member 32B applied to the bent portion 32A. Also, even if the shelf plate 3 is placed on this inclined placement surface 61, the shelf plate 3 is substantially centered due to its own weight and the bent portion 32A of the shelf plate 2, and the placed shelf plate 3 becomes horizontal.

It should be noted that, in an embodiment of the present disclosure, the sliding member 32B is applied so as to cover the flange 32, the bent portion 32A, and the base side surface 35, but as long as the sliding member 32B is provided to at least a portion of any one of the flange 32, the bent portion 32A, and the base side surface 35, it is possible to reduce frictional resistance at the contact part between the shelf plate 3 and the shelf rest 6, which is effective in facilitating sliding of the shelf plate 3.

### Insertion and withdrawal of shelf plate

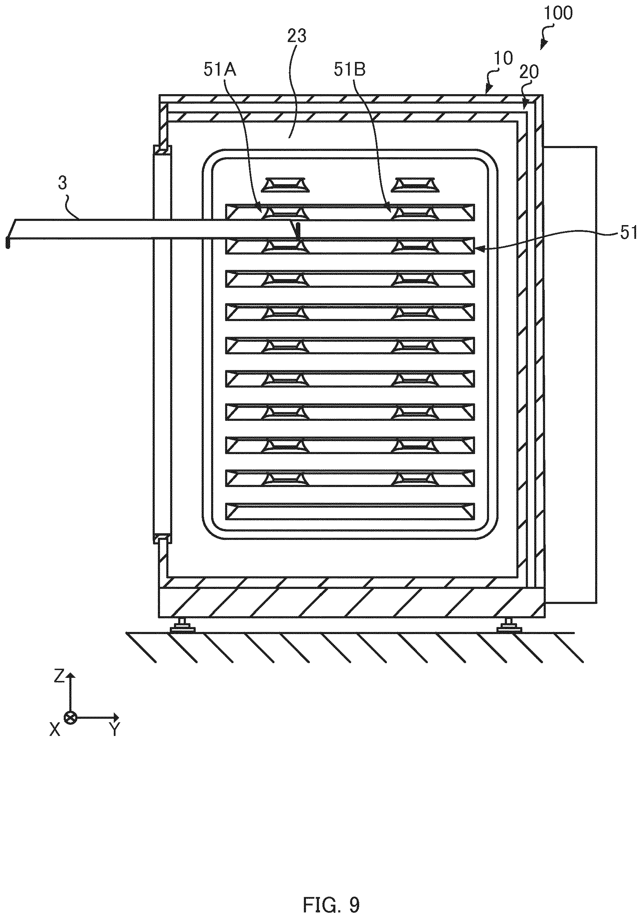

Hereinafter, insertion and withdrawal of the shelf plate according to an embodiment of the present disclosure will be described with reference to FIGS. 8 and 9. FIG. 8 is a top view illustrating the case where the shelf plate 3 has not been inserted or withdrawn in parallel to the shelf rests 5 and 6 according to an embodiment of the present disclosure. FIG. 9 is a side view illustrating the relationship between the shelf plate 3 and the side surface 23 of the inner case according to an embodiment of the present disclosure.

When the outer door 11 and the inner door 21 are opened, the culture apparatus 100 enters a state in which the shelf plate 3 can be inserted into the inner case 20 through the opening 21A. The shelf plate 3 is moved in the front-rear side so as to be placed on shelf rests 51 and 61. An approximately 2 mm gap (gap in the left-right direction) exists between the shelf plate 3 and the side surfaces 22 and 23 of the inner case. Thus, when the shelf plate 3 is moved in the front-rear direction, the shelf plate 3 may not be inserted or withdrawn in a direction parallel to the shelf rests 5 and 6. At this time, the flanges 32 and 33 at the left and right ends of the shelf plate 3 come into contact with the side surfaces 22 and 23 of the inner case 20. This is considered to be caused by a high coefficient of friction at the contact surfaces since the same material is used for the shelf plate 3 and the shelf rests 5 and 6 . In an embodiment of the present disclosure, the lower surface of the base surface 35 of the shelf plate 3 is always in contact with the placement surfaces 51 and 61 of the shelf rests 5 and 6. If the areas, of the shelf plate 3, contacting the shelf rests 5 and 6 are different between the left and right sides, a difference in frictional resistance will be caused between the left and right sides. Thus, even if a worker attempts to insert the shelf plate 3 in parallel to the side surfaces 22 and 23 of the inner case 20, it is difficult to insert the shelf plate 3 in parallel when the frictional resistance is different between the left and right sides. Accordingly, the direction of insertion of the shelf plate 3 becomes inclined relative to the side surfaces 22 and 23 of the inner case 20, and the flanges 32 and 33 on the left and right ends of the shelf plate 3 come into contact with the side surfaces 22 and 23 of the inner case 20.

However, in the culture apparatus 100 according to an embodiment of the present disclosure, the sliding members 32B and 33B, which are made of a material different from the material of the inner case 20, are applied, from the flanges 32 and 33 on the left and right sides to the base side surface, in a range of approximately 10 mm from the end portion on the back surface side (+Y). In other words, the sliding members 32B and 33B are provided on the left and right ends on the front side (+Y) in the traveling direction when the shelf plate 3 is being inserted. For this reason, the coefficient of friction between the base surface 35 of the shelf plate 3 and the placement surfaces 51 and 61 of the shelf rests 5 and 6 is reduced when the shelf plate 3 is inserted, which facilitates sliding of the shelf plate 3. Accordingly, the difference in frictional resistance between the left and right sides decreases, and insertion in a parallel manner is facilitated. Also, even if either one of the back surface sides (+Y) of the flanges 32 and 33 of the shelf plate 3 comes into contact with the side surface 22 or 23 of the inner case 20, it is the sliding member 32B or 33B that comes into contact with the side surface 22 or 23, and therefore the coefficient of friction caused by contact between the same materials decreases, and the shelf plate 3 slides more easily.

Also, when a worker places the shelf plate 3 on the shelf rests 5 and 6, the shelf plate 3 may be inclined relative to the horizontal direction. However, the shelf rest 5 according to an embodiment of the present disclosure has the stoppers 51A and 51B facing the placement surface 51. The shelf rest 6 is also similarly provided with stoppers, but the description thereof is omitted here. The stoppers 51A and 51B are integrally provided to the shelf rest 5 so as to restrict vertical movement of the shelf plate 3 placed on the shelf rest 51. The stoppers 51A and 51B are provided at positions distant from each other along the lengthwise direction (Y axis) of the shelf rest 51.

### Other Embodiments

The first embodiment has been described above as an example of technology disclosed in the present application. However, the technology in the present disclosure is not limited to this, and is also applicable to other embodiments in which appropriate modifications, substitutions, additions, omissions, or the like are made. Also, new embodiments can be achieved by combining the constituent elements described in the above first embodiment.

In view of this, hereinafter, other embodiments will be described collectively.

In the first embodiment, a fluorine coating is used as an example of the sliding members 32B and 33B that are made of a material different from the material of the inner case 20. Fluorine coatings are relatively inexpensive and easy to process during manufacturing. However, the present disclosure is not limited to this. For example, the sliding members 32B and 33B may be a resin coating that uses boron nitride, graphite, or molybdenum disulfide as a main raw material, or a sheet or film having one of these raw materials as its main constituent may be applied. The thickness of the sheet or film is preferably thinner, and it is effective when equal to or smaller than 0.5 mm, and preferably equal to or smaller than 0.2 mm. This is to keep the shelf plate 3 horizontal. Also, it is possible to use a separate removable part that is molded using a resin such as silicone, polyacetal (POM), or polycarbonate (PC). If this separate part is removable, even when using a member not having resistance to sterilizing substances or high-temperature heat in dry heat sterilization, the member can be detached during sterilization,

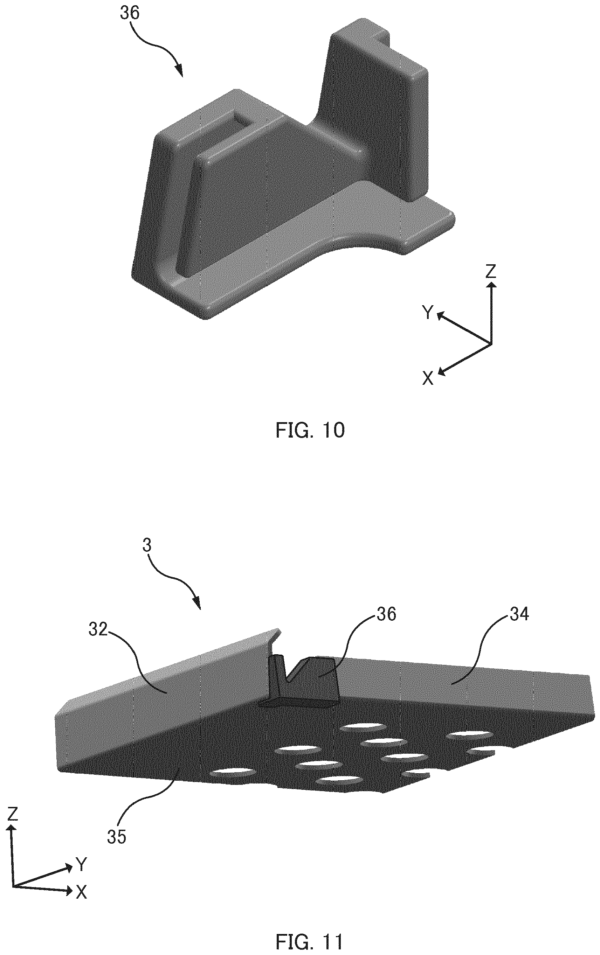

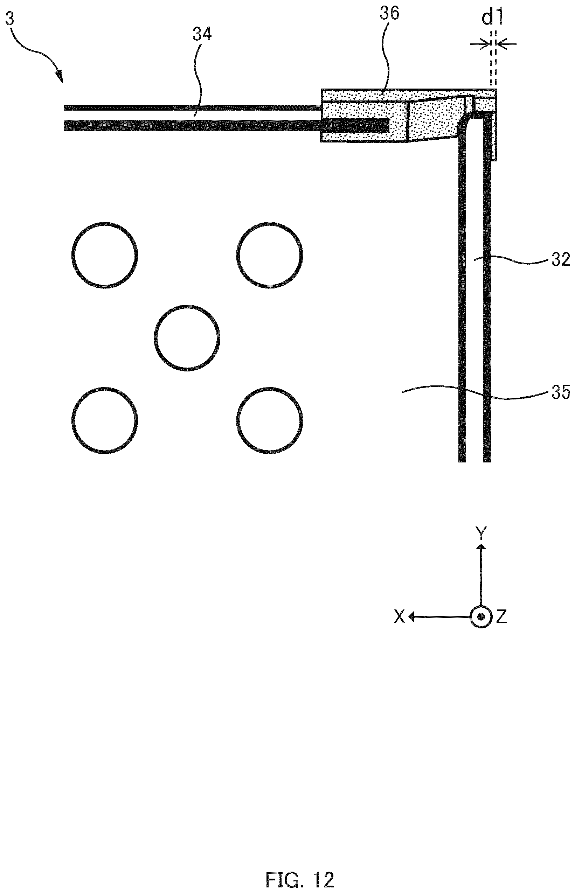

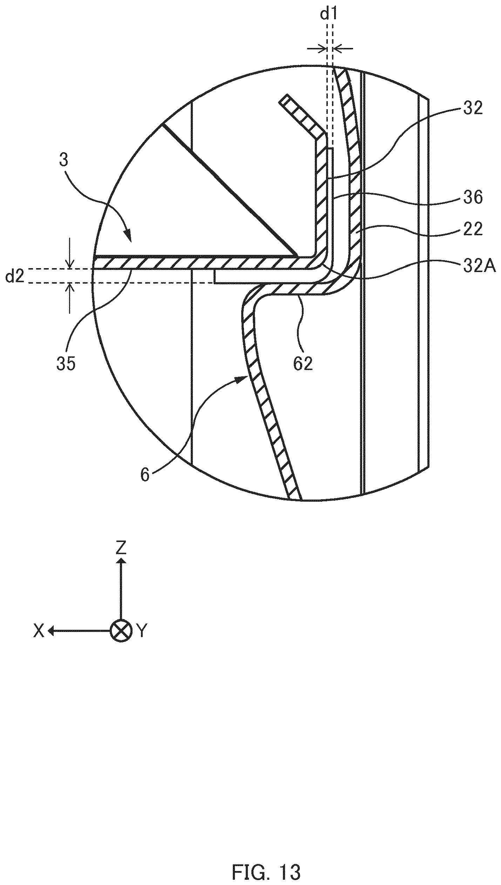

As an example of a culture apparatus that uses separate parts as the sliding members 32B and 33B, the following describes an embodiment using edge guards 36 as separate parts with reference to FIGS. 10 to 13. FIG. 10 is a perspective view illustrating an edge guard 36 in another embodiment. FIG. 11 is an enlarged perspective view illustrating end portions of a back surface flange and a side surface flange to which the edge guard 36 according to this other embodiment has been attached. FIG. 12 is an enlarged top view illustrating the end portions of the back surface flange and the side surface flange to which the edge guard 36 according to this other embodiment has been attached. FIG. 13 is an enlarged cross-sectional view illustrating a portion of contact between the shelf plate 3 and a shelf rest 6 according to another embodiment.

The edge guards 36 are members molded using polyacetal resin. In consideration of the ability to clean the shelf plate 3, the main body of each edge guard 36 and the contact portion between the shelf plate 3 and the edge guard 36 are of shapes in which waste is least likely to accumulate, such as a shape for attachment to the gaps between the flange 34 and the flanges 32 and 33 on the left and right sides of the shelf plate 3. In the state of being attached to the shelf plate 3, the edge guards 36 cover the end portions of the flanges 32 and 33 on the back surface side, portions on the left and right end sides of the flange 34, and portions of the base surface 35. The edge guards 36 are attached to the shelf plate 3 so as to slightly protrude outward (-X and +X) from the flanges 32 and 33 in a view from the front, respectively. In the embodiment illustrated in FIG. 12, a protruding width d1 is 0.2 mm. The width d1 is appropriately determined based on the respective gaps between the flanges 32 and 33 and the side surfaces 22 and 23 of the inner case 20, and is preferably equal to or smaller than 0.5 mm, and further preferably equal to or smaller than 0.2 mm. A thickness d2 of the portion of the edge guard 36 that covers the base surface 35 is effective when equal to or smaller than 1.0 mm, and is further preferably equal to or smaller than 0.5 mm. This is to keep the shelf plate 3 horizontal.

Also, as another embodiment of the sliding members 32B and 33B, in the case where the interior of the culture chamber 21A is sterilized using hydrogen peroxide, it is preferable to use a material resistant to hydrogen peroxide, such as polycarbonate (PC) or polyvinylidene fluoride (PVDF). Also, in the case of sterilizing the interior of the culture chamber 21A by dry heat sterilization, it is preferable to use a material having heat resistance, particularly a material having a heat resistance temperature equal to or larger than 100 degrees, and more preferably a material having a heat resistance temperature equal to or larger than 200 degrees. Examples of materials having a heat resistance temperature equal to or larger than 200 degrees include polyetheretherketone (PEEK), polyether sulfone (PES), polyamide imide (PAI), silicone rubber, and fluororubber.

Also, it is preferable that the sliding members 32B and 33B made of a material different from the material of the inner case 20 are formed using an antibacterial material, such as antibacterial resin or antibacterial glass.

In the first embodiment, as an example of the sliding members 32B and 33B that are made of a material different from the material of the inner case 20, it is described that the sliding members 32B and 33B are applied to the outer surfaces of the flanges 32 and 33 on the left and right sides, the outer surfaces of the bent portions 32A and 33A, and portions of the lower surface of the base side (end) surface 35.

However, it is effective as long as the sliding members 32B and 33B each are provided to at least a portion in any one of each outer surface of the left and right flanges 32 and 33, each outer surface of the bent portions 32A and 33A, and the lower surface of the base side surface 35. In any of these cases, it is effective to be provided to extend from the end portion on the back surface side. This is because sliding of the shelf plate 3 is sometimes disturbed by the end portion on the back surface side being caught on the shelf rest 5 or 6. Here, the phrase "at least a portion" encompasses the case of being provided on the entire surface, and, for example, the sliding members 32B and 33B may be provided on the entirety of the surfaces of the bent portions 32A and 33A or the flanges 32 and 33.

With respect to facilitating sliding of the shelf plate 3, it is effective when the width of the regions of the sliding members 32B and 33B that cover the shelf plate 3 is a width of approximately 2% of the width of the shelf plate 3 in the front-back direction, from the end portion on the back surface side of the shelf plate 3. For this reason, in consideration of the antibacterial property as well, it is preferable that this width is equal to or smaller than 5%, and further preferable that the width is equal to or smaller than 2% since the antibacterial property can be further improved. In order to further improve the slidability of the shelf plate 3, it is effective that this width is in a range of 2% to 10% inclusive.

Also, it is preferable that the vertical width of the regions that cover the flanges 32 and 33 covers the entirety of the surfaces of the flanges 32 and 33. This is because it is possible to restrain damage to the side surfaces 22 and 23 and production of dust, in the case of contact with the side surfaces 22 and 23 of the inner case 20.

It is preferable that the regions of the sliding members 32B and 33B to cover the base surface 35 each are provided to at least a portion, where each of the shelf rests 5 and 6 is to contact, of the surface of the base surface 35. Here, the phrase "at least a portion" encompasses the case of covering the entire surface.

It is preferable that the area of the sliding members 32B and 33B that covers the shelf plate 3 is as large as possible in view of facilitating sliding of the shelf plate 3, but the area is preferably smaller in view of antibacterial property. Depending on the properties of the member, there are cases where a member having a low antibacterial property or none at all, or a member that is antibacterial but high-cost is used. Thus, it is preferable that the area of the regions in which the sliding members 32B and 33B are provided is as small as possible. In particular, since being used in a culture apparatus, the member is preferably an antibacterial member, and is preferably of a shape having less roughness from the view point of cleaning.

Hereinabove, the culture apparatus 100 according to the embodiment described as an example of the present disclosure includes: an inner case 20 having an opening 21A in a front surface and shelf rests 5 and 6 on left and right side surfaces; and a shelf plate 3 to be placed on the shelf rests 5 and 6 and slid in a front-back direction, the shelf plate 3 having a base surface 35 on which a culture is to be placed, flanges 32 and 33 respectively formed by being bent upward, at bent portions 32A and 33A in end portions on left and right sides of the base surface 35, and sliding members 32B and 33B each provided to at least a portion of each of outer surfaces, on a side surface 22, 23 side, of the flanges 32 and 33 on the left and right sides, the sliding members 32B and 33B made of a material different from a material of the inner case 20.

According to this configuration, the sliding members 32B and 33B each are provided to at least a portion of each of the flanges 32 and 33. Thus, even in the case where the shelf plate 3 comes into contact with a side surface of the inner case 20, the frictional resistance between the shelf plate 3 and the side surface of the inner case 20 is reduced. This facilitates sliding of the shelf plate 3 and results in improvement in workability.

Also, the sliding members 32B and 33B each are provided in a range of 10.0 mm from a back surface side on each of the outer surfaces of the flanges 32 and 33 on the left and right sides.

According to this configuration, even in the case where the shelf plate 3 is inclined relative to the side surface 22, 23 of the inner case 20 and either one of the end portions on the back surface side of the flanges 32 and 33 comes into contact with the side surface 22, 23 of the inner case 20, the frictional resistance between the side surface of the inner case 20 and the flange 32, 33 is reduced. This facilitates sliding of the shelf plate 3 and results in improvement in workability. Also, the amount of the material used for the sliding members 32B and 33B can be reduced, which is not only favorable in terms of cost, but also makes it possible to minimize reduction in antibacterial performance of the shelf plate 3 even without using an antibacterial material.

Also, the sliding members 32B and 33B each are provided to at least a portion of each of the outer surfaces, on the side surface 22, 23 side, of the bent portions 32A and 33A on the left and right sides.

According to this configuration, frictional resistance between the bent portions 32A and 33A and the side surfaces 22 and 23 of the inner case 20 or the shelf rests 5 and 6 is reduced. This facilitates sliding of the shelf plate 3 and results in improvement in workability.

Also, the sliding members 32B and 33B each are provided in a range of 10.0 mm from a back surface side on each of the outer surfaces of the bent portions 32A and 33A on the left and right sides.

According to this configuration, even in the case where the shelf plate 3 is inclined relative to the side surface 22, 23 of the inner case 20 and either one of the end portions on the back surface side of the bent portions 32A and 33A comes into contact with the side surface 22, 23 of the inner case 20 or the shelf rest 5, 6, the frictional resistance between the bent portions 32A, 33A and the side surfaces 22, 23 of the inner case 20 or the shelf rests 5, 6 is reduced. This facilitates sliding of the shelf plate 3 and results in improvement in workability. Also, the amount of the material used for the sliding members 32B and 33B can be reduced, which is not only favorable in terms of cost, but also makes it possible to minimize reduction in antibacterial performance of the shelf plate 3 even without using an antibacterial material.

Also, the sliding members 32B and 33B each are provided to at least a portion of a region, where each of the shelf rests 5 and 6 on the left and right sides is to contact, of the lower surface of the base surface 35.

According to this configuration, the frictional resistance between the base surface 35 and the shelf rests 5 and 6 is reduced. This facilitates sliding of the shelf plate 3 and results in improvement in workability.

Also, the sliding members 32B and 33B each are provided in a range of 10.0 mm from a back surface side in a region, where each of the shelf rests 5 and 6 on the left and right sides is to contact, of the lower surface of the base surface 35.

According to this configuration, the frictional resistance between the end portions on the back surface side of the base surface 35 and the shelf rests 5 and 6 is reduced This facilitates sliding of the shelf plate 3 and results in improvement in workability. Also, the amount of the material used for the sliding members 32B and 33B can be reduced, which is not only favorable in terms of cost, but also makes it possible to minimize reduction in the antibacterial performance of the shelf plate 3 even without using an antibacterial material.

Also, the sliding members 32B and 33B are removable from or integrated with the shelf plate 3.

According to this configuration, if the sliding members 32B and 33B are removable from the shelf plate 3, they can be removed when performing sterilization with hydrogen peroxide or by dry heat sterilization. This not only increases the number of types of culture apparatus to which the present disclosure is applicable, but also increases the number of materials that can be selected as a material for the sliding members 32B and 33B. Also, when the sliding members 32B and 33B are integrated with the shelf plate 3, it is possible to save worker's time and effort for attachment and removal.

Also, the sliding members 32B and 33B are made of a material having heat resistance to 100 degrees or more.

According to this configuration, the sliding members 32B and 33B do not change in physical property at 100 degrees or more, and thus can also be applied to a culture apparatus in which the interior 2A of the inner case 20 is sterilized by dry heat sterilization at 100 degrees or more. It is further preferable that the sliding members 32B and 33B are made of a material having heat resistance equal to or larger than 200 degrees, since they can also be applied to a culture apparatus in which dry heat sterilization at 200 degrees or more is carried out. For example, it is preferable that the sliding members 32B and 33B are made of a material containing polyetheretherketone (PEEK), polyether sulfone (PES), polyamide imide (PAI), silicone rubber, or fluororubber, since the sliding members 32B and 33B should have heat resistance equal to or larger than 200 degrees.

Also, the sliding members 32B and 33B may be made of an antibacterial material.

According to this configuration, an antibacterial material is used for the sliding members 32B and 33B. This makes it possible to provide a culture apparatus that facilitates sliding of the shelf plate 3 and has a high antibacterial property.

Also, the sliding members 32B and 33B may be resin members.

According to this configuration, frictional resistance between the shelf plate 3 and the side surfaces 22, 23 of the inner case 20 or the shelf rests 5, 6 is reduced and sliding of the shelf plate 3 is facilitated, as well as processing and attachment of the sliding members 32B and 33B are also facilitated.

Also, the sliding members 32B and 33B may be fluorine coatings.

According to this configuration, frictional resistance between the shelf plate 3 and the side surfaces 22, 23 of the inner case 20 or the shelf rests 5, 6 is reduced and sliding of the shelf plate 3 is facilitated, as well as processing is facilitated. This enables application of coatings to a minimum required range, thereby being able to minimize reduction in the antibacterial property.

Also, the sliding members 32B and 33B may be made of a material resistant to hydrogen peroxide.

According to this configuration, the present disclosure is applicable to a culture apparatus in which sterilization is performed using hydrogen peroxide. For example, it is preferable that the sliding members 32B and 33B are made of a material containing polycarbonate (PC) or polyvinylidene fluoride (PVDF).

Embodiments have been described above as examples of the present disclosure. The accompanying drawings and detailed description have been provided for this purpose. The embodiments described above are to facilitate the understanding of the present disclosure, and are not to be construed as limiting the present disclosure.

Also, the constituent elements included in the accompanying drawings and the detailed description may include not only constituent elements that are essential for solving the problem, but also constituent elements that are not essential for solving the problem, in order to illustrate examples of the techniques. Thus, these non-essential constituent elements should not be immediately found to be essential constituent elements based on the fact that they are included in the accompanying drawings or detailed description.

### [Reference Signs List]

100 culture apparatus, 20 inner case, 2A culture chamber, 10 outer case, 20 inner case, 21A opening, 22,23 side plate, 24 back plate, 25 bottom plate, 26 top plate, 3 shelf plate, 31, 32, 33, 34 flange, 32A, 33A bent portion, 32B, 33B sliding member, 35 base surface, 5,6 shelf rest

## Claims

1. A culture apparatus comprising:
an inner case (20) having an opening (21A) in a front surface and stainless-steel shelf rests (6) on left and right side surfaces (22, 23); and
a stainless-steel shelf plate (3) to be placed on the shelf rests and slid in a front-back direction,
the shelf plate having
a base surface (35) on which a culture is to be placed,
flanges (32, 33) respectively formed by being bent upward, at bent portions(32A, 33A), in end portions on left and right sides of the base surface, and
sliding members (32B, 33B, 36) respectively provided: to outer surfaces of the flanges on the left and right sides in a range of 10 mm from a back surface side; and/or on each of the outer surfaces of the bent portions, on the left and right sides; and/or in a region, where each of the shelf rests on the left and right sides is to contact, of the lower surface of the base surface,
wherein the sliding members are made of a material different from a material of the inner case,
wherein the sliding members are fluorine coatings or constituted by: a resin coating using boron nitride, graphite, or molybdenum disulfide as a main raw material; or a sheet or film having one of fluorine, boron nitride, graphite, and molybdenum disulfide as its main constituent,
and wherein the sliding members are made of a material resistant to hydrogen peroxide and/or have heat resistance to 100 degrees or more.

2. The culture apparatus according to claim 1, wherein the sliding members (32, 33) are constituted by a sheet or film having one of boron nitride, graphite and molybdenum disulfide as its main constituent, and each have a thickness equal to or smaller than 0.2 mm.

3. The culture apparatus according to claim 1, wherein the sliding members (32B, 33B, 36) are each provided on the outer surfaces of the flanges (32, 33) on the left and right sides, the outer surfaces of the bent portions (32A, 33A), and portions of the lower surface of the base side surface (35).

4. The culture apparatus according to claim 1, wherein the shelf rests(6) each further include a curved portion provided in a portion connecting a placement surface (61) on which the shelf plate (3) is to be placed and each of the side surfaces (22) of the inner case (20), and the sliding members (32, 33, 36) each are provided to each of the outer surfaces of the bent portions on the left and right sides.

5. The culture apparatus according to claim 2 or 4, wherein the curved portion and each of the sliding members (32, 33, 36) provided to the bent portions are configured to come into contact with each other.

6. The culture apparatus according to any one of claims 1 to 5, wherein the sliding members (32B, 33B, 36) are removable from or integrated with the shelf plate.

7. The culture apparatus according to any one of claims 1 to 6, wherein the sliding members (32B, 33B) are made of a material having heat resistance to 200 degrees or more.

8. The culture apparatus according to claim 1 or 7, wherein the sliding members (32B, 33B, 36) are made of a material containing polyetheretherketone (PEEK), polyether sulfone (PES), polyamide imide (PAI), silicone rubber, or fluororubber.

9. The culture apparatus according to any one of claims 1 to 8, wherein the sliding members (32B, 33B, 36) are made of an antibacterial material.

10. The culture apparatus according to any one of claims 1 to 9, wherein the sliding members (32B, 33B, 36) are resin members.

11. The culture apparatus according to claim 10, wherein the sliding members (32B, 33B, 36) are configured with a resin molding containing silicone, polyacetal (POM), or polycarbonate (PC).

12. The culture apparatus according to claim 1, wherein the sliding members (32B, 33B, 36) are made of a material containing polycarbonate (PC) or polyvinylidene fluoride (PVDF).

13. The culture apparatus according to any one of claims 1 to 12, wherein a width of the regions of the sliding members (32B, 33B, 36) that cover the shelf plate (3) in the front-back direction is in a range of 2% to 10% of the width of the shelf plate, from the end portion on the back surface side of the shelf plate.

## Patentansprüche

1. Kulturvorrichtung, die umfasst:
ein inneres Gehäuse (20) mit einer Öffnung (21A) in einer vorderen Fläche und Fachauflagen (6) aus rostfreiem Stahl an einer linken und einer rechten Seitenfläche (22, 23); sowie
eine Auflageplatte (3) aus rostfreiem Stahl, die auf die Fachauflagen aufgelegt und nach vorne und hinten verschoben wird,
wobei die Auflageplatte aufweist:
eine Grundfläche (35), auf der eine Kultur positioniert wird,
Flansche (32, 33), die jeweils durch Biegen nach oben an Biege-Abschnitten (32A, 33A) ausgebildet werden, an Endabschnitten an der linken und der rechten Seite der Grundfläche, sowie
Gleitelemente (32B, 33B, 36), die jeweils an Außenflächen der Flansche an der linken und der rechten Seite in einem Bereich von 10 mm von der Seite einer hinteren Fläche und/oder an jeder der Außenflächen der Biege-Abschnitte an der linken und der rechten Seite; und/oder in einem Bereich der unteren Fläche der Grundfläche vorhanden sind, mit dem jede der Fachauflagen an der linken und der rechten Seite in Kontakt kommt,
wobei die Gleitelemente aus einem Material bestehen, das sich von einem Material desinneren Gehäuses unterscheidet,
die Gleitelemente Beschichtungen aus Fluor sind oder durch eine Beschichtung aus Kunststoff, für die Bornitrid, Graphit oder Molybdändisulfid als ein Haupt-Ausgangs-material eingesetzt wird; oder eine Folie oder einen Film gebildet werden, die/der Fluor, Bornitrid, Graphit oder Molybdändisulfid als seinen Hauptbestandteil aufweist,
und die Gleitelemente aus einem gegen Wasserstoffperoxid beständigen Material bestehen und/oder eine Wärmebeständigkeit bis 100 ° oder darüber haben.

2. Kulturvorrichtung nach Anspruch 1, wobei die Gleitelemente (32, 33) aus einer Folie oder einem Film bestehen, die/der Bornitrid, Graphit oder Molybdändisulfid als seinen Hauptbestandteil aufweist, und sie jeweils eine Dicke von 0,2 mm oder weniger haben.

3. Kulturvorrichtung nach Anspruch 1, wobei die Gleitelemente (32B, 33B, 36) jeweils an den Außenflächen der Flansche (32, 33) an der linken und der rechten Seite, den Außenflächen der Biege-Abschnitte (32A, 33A) sowie Abschnitten der unteren Fläche der Grundfläche (35) vorhanden sind.

4. Kulturvorrichtung nach Anspruch 1, wobei die Fachauflagen (6) des Weiteren jeweils einen gekrümmten Abschnitt aufweisen, der in einem Abschnitt vorhanden ist, der eine Auflagefläche (61), auf die die Auflageplatte (3) aufgelegt wird, und jede der Seitenflächen (22) des inneren Gehäuses (20) verbindet, und die Gleitelemente (32, 33, 36) jeweils an jeder der Außenflächen der Biege-Abschnitte an der linken und der rechten Seite vorhanden sind.

5. Kulturvorrichtung nach Anspruch 2 oder 4, wobei der gekrümmte Abschnitt und jedes der Gleitelemente (32, 33, 36), die an den gekrümmten Abschnitten vorhanden sind, so eingerichtet sind, dass sie miteinander in Kontakt kommen.

6. Kulturvorrichtung nach einem der Ansprüche 1 bis 5, wobei die Gleitelemente (32B, 33B, 36) von der Auflageplatte abgenommen werden können oder in diese integriert sind.

7. Kulturvorrichtung nach einem der Ansprüche 1 bis 6, wobei die Gleitelemente (32B, 33B) aus einem Material mit einer Wärmebeständigkeit von 200 ° oder darüber bestehen.

8. Kulturvorrichtung nach Anspruch 1 oder 7, wobei die Gleitelemente (32B, 33B, 36) aus einem Material bestehen, das Polyetheretherketon (PEEK), Polyethersulfon (PES), Polyamidimid (PAI), Silikonkautschuk oder Fluorkautschuk enthält.

9. Kulturvorrichtung nach einem der Ansprüche 1 bis 8, wobei die Gleitelemente (32B, 33B, 36) aus einem antibakteriellen Material bestehen.

10. Kulturvorrichtung nach einem der Ansprüche 1 bis 9, wobei die Gleitelemente (32B, 33B, 36) Elemente aus Kunststoff sind.

11. Kulturvorrichtung nach Anspruch 10, wobei die Gleitelemente (32B, 33B, 36) als ein Kunststoff-Formteil ausgeführt sind, das Silikon, Polyacetal (POM) oder Polycarbonat (PC) enthält.

12. Kulturvorrichtung nach Anspruch 1, wobei die Gleitelemente (32B, 33B, 36) aus einem Material bestehen, das Polycarbonat (PC) oder Polyvinylidenfluorid (PVDF) enthält.

13. Kulturvorrichtung nach einem der Ansprüche 1 bis 12, wobei eine Breite der Bereiche der Gleitelemente (32B, 33B, 36), die die Auflageplatte (3) in der Längsrichtung abdecken, in einem Bereich von 2 % bis 10 % der Breite der Auflageplatte von dem Endabschnitt an der Seite der hinteren Fläche der Auflageplatte aus liegt.

## Revendications

1. Appareil de culture comprenant :
une enveloppe intérieure (20) ayant une ouverture (21A) dans une surface avant et des supports à étagère (6) en acier inoxydable sur des surfaces latérales gauche et droite (22, 23) ; et
une plaque étagère (3) en acier inoxydable destinée à être placée sur les supports à étagère et coulissée suivant un sens avant-arrière,
la plaque étagère ayant
une surface de base (35) sur laquelle une culture doit être placée,
des rebords (32, 33) formés respectivement par pliage vers le haut, au niveau de parties pliées (32A, 33A), dans des parties d'extrémité sur des côtés gauche et droit de la surface de base, and
des éléments de coulissement (32B, 33B, 36) fournis respectivement : sur des surfaces extérieures des rebords sur les côtés gauche et droit sur une plage de 10 mm à partir d'un côté de surface arrière ; et/ou sur chacune des surfaces extérieures des parties pliées, sur les côtés gauche et droit ; et/ou dans une région de la surface inférieure de la surface de base où chacun des supports à étagère sur les côtés gauche et droit doit entrer en contact,
les éléments de coulissement étant constitués d'un matériau différent d'un matériau de l'enveloppe intérieure,
les éléments de coulissement étant des revêtements fluorés ou constitués par : un revêtement en résine utilisant du nitrure de bore, du graphite, ou du disulfure de molybdène comme matière première principale ; ou une feuille ou un film ayant un élément parmi le fluor, le nitrure de bore, le graphite et le disulfure de molybdène en tant que constituant principal,
et les éléments de coulissement étant constitués d'un matériau résistant au peroxyde d'hydrogène e/ou ayant une résistance à la chaleur de 100 degrés ou plus.

2. Appareil de culture selon la revendication 1, dans lequel les éléments de coulissement (32, 33) sont constitués par une feuille ou un film ayant un élément parmi le fluor, le nitrure de bore, le graphite et le disulfure de molybdène en tant que constituant principal, et ils ont chacun une épaisseur inférieure ou égale à 0,2 mm.

3. Appareil de culture selon la revendication 1, dans lequel les éléments de coulissement (32B, 33B, 36) sont situés chacun sur les surfaces extérieures des rebords (32, 33) sur les côtés gauche et droit, les surfaces extérieures des parties pliées (32A, 33A) et des parties de la surface inférieure de la surface latérale de base (35).

4. Appareil de culture selon la revendication 1, dans lequel les supports à étagère (6) incluent chacun, en outre, une partie incurvée située dans une partie reliant une surface de placement (61) sur laquelle doit être placée la plaque étagère (3) et chacune des surfaces latérales (22) de l'enveloppe intérieure (20), et les éléments de coulissement (32, 33, 36) sont situés chacun sur chacune des surfaces extérieures des parties pliées sur les côtés gauche et droit.

5. Appareil de culture selon la revendication 2 ou 4, dans lequel la partie incurvée et chacun des éléments de coulissement (32, 33, 36) situé sur les parties pliées sont configurés pour venir en contact l'un avec l'autre.

6. Appareil de culture selon l'une quelconque des revendications 1 à 5, dans lequel les éléments de coulissement (32B, 33B, 36) sont démontables ou intégrés à la plaque étagère.

7. Appareil de culture selon l'une quelconque des revendications 1 à 6, dans lequel les éléments de coulissement (32B, 33B) sont constitués d'un matériau ayant une résistance à la chaleur de 200 degrés ou plus.

8. Appareil de culture selon la revendication 1 ou 7, dans lequel les éléments de coulissement (32B, 33B, 36) sont constitués d'un matériau contenant de la polyétheréthercétone (PEEK), du polyéther sulfone (PES), du polyamide-imide (PAI), du caoutchouc à la silicone, ou un caoutchouc fluoré.

9. Appareil de culture selon l'une quelconque des revendications 1 à 8, dans lequel les éléments de coulissement (32B, 33B, 36) sont constitués d'un matériau antibactérien.

10. Appareil de culture selon l'une quelconque des revendications 1 à 9, dans lequel les éléments de coulissement (32B, 33B, 36) sont des éléments en résine.

11. Appareil de culture selon la revendication 10, dans lequel les éléments de coulissement (32B, 33B, 36) sont configurés avec un moulage en résine contenant de la silicone, du polyacétal (POM), ou du polycarbonate (PC).

12. Appareil de culture selon la revendication 1, dans lequel les éléments de coulissement (32B, 33B, 36) sont constitués d'un matériau contenant du polycarbonate (PC) ou du fluorure de polyvinylidène (PVDF).

13. Appareil de culture selon l'une quelconque des revendications 1 à 12, dans lequel une largeur des régions des éléments de coulissement (32B, 33B, 36) qui couvrent la plaque étagère (3) dans le sens avant-arrière est dans une plage de 2 % à 10 % de la largeur de la plaque étagère, depuis la partie d'extrémité sur le côté de surface arrière de la plaque étagère.
